# EUROPEAN PATENT SPECIFICATION

(11) **EP 0 527 036 B1**
(45) Date of publication and mention of the grant of the patent: **06.11.1996**
(21) Application number: 92307122.9
(22) Date of filing: 04.08.1992
(51) Int. Cl.: C07D 261/08, A01N 43/80

(54) **4-Benzoylisoxazole derivatives and their use as herbicides**
4-Benzoylisoxazol-Derivate und ihre Anwendung als Herbizide
Dérivés de 4-benzoylisoxazole et leur utilisation comme herbicides

(30) Priority: 05.08.1991 GB 9116834
(43) Date of publication of application: 10.02.1993
(73) Proprietor: RHONE-POULENC AGRICULTURE LTD., Ongar, Essex CM5 0HW (GB)
(72) Inventor: Cain, Paul Alfred, c/o Research Station, Ongar, Essex CM5 0HW (GB); Cramp, Susan Mary, c/o Research Station, Ongar, Essex CM5 0HW (GB); Little, Gillian Mary, c/o Research Station, Ongar, Essex CM5 0HW (GB); Luscombe, Brian Malcolm, c/o Research Station, Ongar, Essex CM5 0HW (GB)
(74) Representative: Bentham, Stephen

(56) References cited:
- EP-A- 487 357
- EP-A- 0 418 175

## Description

This invention relates to novel 4-benzoyl isoxazole derivatives, compositions containing them and their use as herbicides. The present invention provides 4-benzoyl isoxazoles of general formula (I): 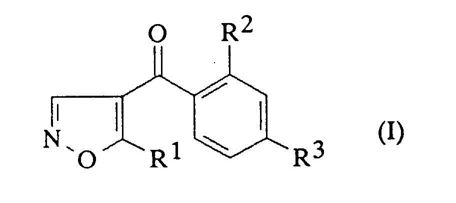 wherein:
R¹ represents cyclopropyl;
R² represents -S(O)ₙR;
R³ represents chlorine, bromine or trifluoromethyl;
R represents methyl; and
n represents 2;
which possess valuable herbicidal properties.
The present invention therefore provides the following compounds:
A) 4-(4-chloro-2-methylsulphonylbenzoyl)-5-cyclopropylisoxazole;
B) 5-cydopropyl-4-(2-methylsulphonyl-4-trifluoromethylbenzoyl) isoxazole; and
C) 4-(4-bromo-2-methylsulphonylbenzoyl)-5-cyclopropylisoxazole. The letters A to C are assigned to the above compounds for reference and identification hereafter.

EP-A-0418175 discloses a class of 4-benzoyl isoxazole derivatives useful as herbicides but contains no specific disclosure of the compounds of the present invention.

EP-A-0487357, which is a document that falls under Article 54(3) EPC, also discloses 4-benzoyl isoxazole derivatives useful as herbicides.

The compounds of the invention show unexpected and remarkably high herbicidal activity in comparison with known compounds against important weed species including foxtail (Setaria viridis and Setaria faberii), barnyard grass (Echinochloa crus-galli), crabgrass (Digitaria sanguinalis) and shattercane (Sorphum bicolor).

Compounds of general formula (I) may be prepared by the application or adaptation of known methods (i.e. methods heretofore used or described in the literature), for example as hereinafter described.

In the following description where symbols appearing in formulae are not specifically defined, it is to be understood that they are "as hereinbefore defined" in accordance with the first definition of each symbol in the specification.

It is to be understood that in the descriptions of the following processes the sequences may be performed in different orders, and that suitable protecting groups may be required to achieve the compounds sought.

According to a feature of the present invention compounds of general formula (I) may be prepared by the reaction of a compound of general formula (II): 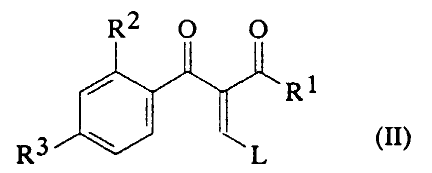 wherein L is a leaving group, with a salt of hydroxylamine.
Hydroxylamine hydrochloride is generally preferred. Generally L is O-alkyl or N,N-dialkylamino, for example ethoxy or dimethylamino. The reaction is generally carried out in a solvent such as ethanol or acetonitrile, optionally in the presence of a base or acid acceptor such as triethylamine or sodium acetate.

According to a further feature of the present invention compounds of general formula (I) may be prepared by the oxidation of compounds of general formula (III): 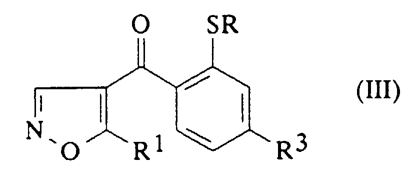 to convert the sulphenyl group to a sulphonyl group. The oxidation of the sulphenyl group is generally carried out using for example 3-chloroperoxybenzoic acid in an inert solvent such as dichloromethane at a temperature from -40°C to O°C.

According to a further feature of the present invention compounds of general formula (I) may be prepared by the reaction of a compound of general formula (IV): 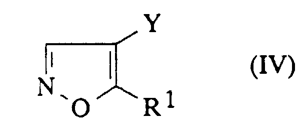 wherein Y represents a carboxy group or a reactive derivative thereof (such as a carboxylic acid chloride or carboxylic ester), or a cyano group, with an appropriate organometallic reagent such as a Grignard reagent or an organolithium reagent. The reaction is generally carried out in an inert solvent such as ether or tetrahydrofuran at a temperature from 0°C to the reflux temperature of the mixture.

Intermediates in the preparation of compounds of general formula (I) are prepared by the application or adaptation of known methods.

Compounds of general formula (III) may be prepared by the reaction of a compound of general formula (V): 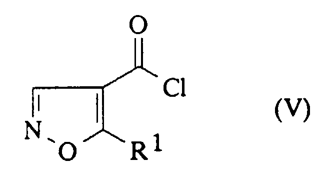 with a compound of general formula (VI):

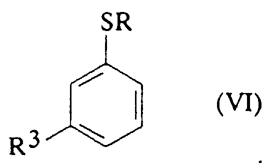

The reaction is generally carried out in the presence of a Lewis acid catalyst such as aluminium chloride at a temperature between room temperature and 100°C.

Compounds of general formula (III) may also be prepared by the reaction of a compound of general formula (VII): 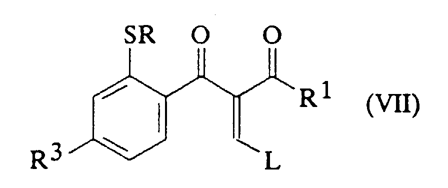 wherein L is a leaving group, with a salt of hydroxylamine.
Hydroxylamine hydrochloride is generally preferred. Generally L is O-alkyl or N,N-dialkylamino, for example ethoxy or dimethylamino. The reaction is generally carried out in a solvent such as ethanol or acetonitrile, optionally in the presence of a base or acid acceptor such as triethylamine or sodium acetate.

Compounds of general formula (III) may also be prepared by the reaction of a compound of general formula (IV) wherein Y represents a carboxy group or a reactive derivative thereof (such as a carboxylic acid chloride or carboxylic ester), or a cyano group, with an appropriate organometallic reagent such as a Grignard reagent or an organolithium reagent. The reaction is generally carried out in an inert solvent such as ether or tetrahydrofuran at a temperature from 0°C to the reflux temperature of the mixture.

Compounds of general formula (II) may be prepared by the reaction of compounds of general formula (VIII): 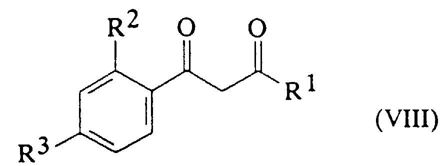 with either a trialkyl orthoformate or a dimethylformamide dialkyl acetal. Generally triethyl orthoformate or dimethylformamide dimethyl acetal are used. The reaction with a trialkyl orthoformate is generally carried out in the presence of acetic anhydride at the reflux temperature of the mixture and the reaction with a dimethylformamide dialkyl acetal is carried out optionally in the presence of an inert solvent at a temperature from room temperature to the reflux temperature of the mixture.

Compounds of general formula (VII) may be prepared by the reaction of compounds of general formula (IX): 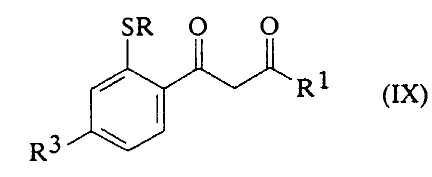 with a trialkyl orthoformate or a dimethylformamide dialkyl acetal. The reaction may be carried out as hereinbefore described for the preparation of a compound of formula (II) from a compound of formula (VIII).

The preparation of intermediates of general formulae (IV), (V), (VI), (VIII) and (IX) is described extensively in the literature.

The following Examples illustrate the preparation of compounds of general formula (I) and the Reference Examples illustrate the preparation of intermediates of the invention. In the present specification b.p. means boiling point; m.p. means melting point. Where the letters NMR appear, the characteristics of the proton nuclear magnetic resonance spectrum follow.

### EXAMPLE 1

### Compounds A, B and C

Sodium acetate (1.93g) was added to a mixture of 1-(4-chloro-2-methylsulphonyl-phenyl)-3-cyclopropyl-2-ethoxymethylenepropan-1,3-dione (8.4g) and hydroxylamine hydrochloride (1.64g) in ethanol with stirring. The mixture was stirred at room temperature overnight. It was evaporated and the residue was dissolved in ethyl acetate, washed with water, dried (anhydrous magnesium sulphate) and filtered. The filtrate was evaporated to dryness and the residue was recrystallized from ether to give 4-(4-chloro-2-methylsulphonylbenzoyl)-5-cyclopropylisoxazole (4.4g) as an off-white solid, m.p. 184-185°C.

By proceeding in a similar manner the following compounds of general formula (I) were prepared from the appropriately substituted starting materials:

| Compound No | R¹ | R² | R³ | m.p. |
|---|---|---|---|---|
| B | Cyclopropyl | SO₂Me | CF₃ | 138-138.5°C |
| C | Cyclopropyl | SO₂Me | Br | 201-202°C |

### REFERENCE EXAMPLE 1

A mixture of 1-(4-chloro-2-methylsulphonylphenyl)-3-cyclopropylpropan-1,3-dione (7.1g) and triethylorthoformate (6.9g) in acetic anhydride was stirred and heated at reflux for 2 hours. The mixture was evaporated to dryness, toluene added and the solution was re-evaporated to give 1-(4-chloro-2-methylsulphonylphenyl)-3-cyclopropyl-2-ethoxymethylenepropan-1,3-dione (8.4g) as a red gum which was not purified further.

By proceeding in a similar manner the following compounds were prepared from the appropriately substituted starting materials:

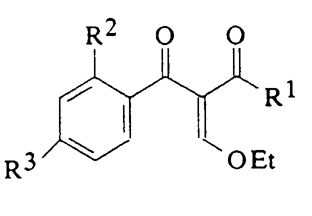

| **R**^{**1**} | **R**^{**2**} | **R**^{**3**} |
|---|---|---|
| Cyclopropyl | SO₂Me | CF₃ |
| Cyclopropyl | SO₂Me | Br |

### REFERENCE EXAMPLE 2

A mixture of t-butyl 2-(4-chloro-2-methylsulphonylbenzoyl)-3-cyclopropyl-3-oxopropionate (9.5g) and 4-toluenesulphonic acid (1.5g) in toluene was stirred and heated at reflux for 3 hours. After cooling, the mixture was washed with water, dried (anydrous magnesium sulphate) and filtered. The filtrate was evaporated to dryness to give 1-(4-chloro-2-methylsulphonylphenyl)-3-cyclopropylpropan-1,3-dione, (7.1g) as an orange gum, NMR (CDCl₃): 0.8-1.2 (m, 4H), 1.5-1.9 (m, 1H), 3.3 (s, 3H), 5.8 (s,1H), 7.3-7.6 (m,2H), 7.9 (s,1H).

By proceeding in a similar manner the following compounds were prepared from the appropriately substituted starting materials:

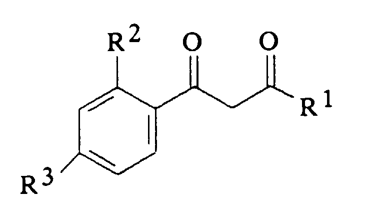

| **R**^{**1**} | **R**^{**2**} | **R**^{**3**} | **NMR** |
|---|---|---|---|
| Cyclopropyl | SO₂Me | CF₃ | NMR(CDCl₃):0.8-1.4(m,4H), 1.5-1.8 (m,1H), 3.3(s,3H),5.85(s,1H), 7.5 (d,1H), 7.8(d,1H), 8.2(s,1H) |
| Cyclopropyl | SO₂Me | Br | NMR(CDCl₃): 0.9-1.35(m,4H), 1.5-1.9(m,1H), 3.45(s,3H), 6.0(s,1H), 7.5(d,1H),7.95(d,1H), 8.4(s,1H). |

### REFERENCE EXAMPLE 3

Carbon tetrachloride (2ml) was added to a mixture of magnesium (0.57g) and t-butyl 3-cyclopropyl-3-oxopropionate (4.36g) in methanol. The mixture was stirred for 0.5 hours. It was evaporated to dryness and the residue was dissolved in toluene. The solution was evaporated to dryness and the residue was suspended in acetonitrile. 4-Chloro-2-methylsulphonylbenzoyl chloride (6.0g) was added and the mixture was stirred for 3 hours. It was evaporated to dryness and the residue was dissolved in ethyl acetate. The mixture was washed with aqueous hydrochloric acid (2M), water, dried (anhydrous magnesium sulphate) and filtered. The filtrate was evaporated to dryness to give t-butyl 2-(4-chloro-2-methylsulphonylbenzoyl)-3-cyclopropyl-3-oxopropionate (9.6g) as a brown oil which was not further purified.

By proceeding in a similar manner the following compounds were prepared from the appropriately substituted starting materials:

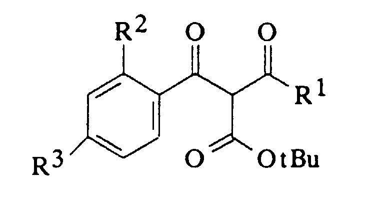

| R¹ | R² | R³ |
|---|---|---|
| Cyclopropyl | SO₂Me | CF₃ |
| Cyclopropyl | SO₂Me | Br |

Benzoyl chlorides were prepared by heating the appropriately substituted benzoic acids at reflux with thionyl chloride for 3 hours. The excess thionyl chloride was removed by evaporation and the benzoyl chlorides thus obtained were used directly without further purification.

### REFERENCE EXAMPLE 4

Potassium permanganate (316g) was added with stirring to a suspension of 4-bromo-2-methylsulphenyltoluene (90.5g) in water whilst maintaining the mixture at reflux. The mixture was stirred and heated at reflux for 3 hours. The mixture was filtered and the residue was washed with hot water. The filtrate was cooled to room temperature and extracted with ethyl acetate. The aqueous solution was acidified to pH 1, saturated with sodium chloride and extracted with ethyl acetate. The organic layer was washed with water, dried (anhydrous magnesium sulphate) and filtered. The filtrate was evaporated to dryness to give 4-bromo-2-methylsulphonylbenzoic acid (44.6g) as a light brown solid, m.p. 220-220.5°C.

By proceeding in a similar manner the following compound was prepared from the appropriately substituted starting materials:
4-chloro-2-methylsulphonylbenzoic acid; NMR (CDCl₃ + DMSO-d₆): 3.35 (s,3H), 7.5-7.8(m,2H), 7.9 (s,1H), 8.2-8.6 (bs,1H);

### REFERENCE EXAMPLE 5

Hydrogen peroxide (30%) was added to a cooled solution of 2-methylsulphenyl-4-trifluoromethylbenzoic acid (6.0g) and acetic anhydride(3.6ml) in acetic acid at 10°C. The mixture was allowed to warm slowly to room temperature and stirred for 0.5 hours. It was stirred and heated at 65°C for 3 hours. After cooling the mixture was poured into ice and extracted with ether. The organic layer was washed with water, aqueous ferrous sulphate solution, dried (anhydrous magnesium sulphate) and filtered. The filtrate was evaporated to dryness to give 2-methylsulphonyl-4-trifluoromethylbenzoic acid (5.54g) as a white solid, m.p. 155.5-156.5°C.

### REFERENCE EXAMPLE 6

n-Butyllithium (2.5M solution in hexane; 25 ml) was added dropwise under an inert atmosphere to a stirred solution 4-bromo-3-methylsulphenylbenzotrifluoride (16.4g) in ether at -70°C for 2 hours and then poured onto solid carbon dioxide pellets. The mixture was stirred for 10 minutes and aqueous hydrochloric acid was added. The layers were separated and the aqueous layer was extracted with ether. The combined organic layers were washed with water, dried (anhydrous magnesium sulphate) and filtered. The filtrate was evaporated and the residue was triturated with cyclohexane and filtered to give 2-methylsulphenyl-4-trifluoromethylbenzoic acid (12.4g) as a white solid, NMR (CDCl₃) + DMSO-d₆): 2.45 (s,3H), 7.2(d,1H), 7.3(s,1H), 8.0 (d,1H), 10.7-11.1 (bs,1H).

### REFERENCE EXAMPLE 7

t-Butyl nitrite (4ml) was added to a mixture of 5-chloro-2-methylaniline (4g) and dimethyl disulphide (26.3g) in chloroform. After the reaction started t-butyl nitrite (17.7ml) and 5-chloro-2-methylaniline (16g) were added simultaneously. The mixture was stirred at room temperature for 2 hours and left to stand overnight. The mixture was washed with water, aqueous hydrochloric acid (2m), water, dried (anhydrous magnesium sulphate) and filtered. The filtrate was evaporated to dryness to give 4-chloro-2-methylsulphenyltoluene (24.6g) as a red oil, NMR (CDCl₃): 2.2 (s,3H), 6.85 (s,2H), 7.0 (s,1H).

By proceeding in a similar manner the following compound was prepared from the appropriately substituted starting materials:
4-bromo-3-methylsulphenylbenzotrifluoride, b.p. 84-88°C at 267 Pa (2mm Hg).

### REFERENCE EXAMPLE 8

A cooled solution of sodium nitrite (5.8g) in concentrated sulphuric acid (50 ml) was added dropwise to a stirred solution of 4-methyl-3-methylsulphenylaniline (12.8g) in glacial acetic acid at 20°C. The resulting suspension was added to a mixture of copper (I) bromide (12g), aqueous hydrobromic acid (48-50%) and ice. The mixture was stirred at room temperature for 3 hours then diluted with water and extracted with ethyl acetate. The organic layer was washed with water, aqueous sodium hydroxide (2m), dried (anhydrous magnesium sulphate) and filtered. The filtrate was evaporated to dryness. The residue was triturated with hot cyclohexane and filtered. The filtrate was evaporated to dryness to give 4-bromo-2-methylsulphenyltoluene (8.6g) as a brown oil, NMR (CDCl₃): 2.15(s,3H), 2.2(s,3H), 6.5-7.1 (m,3H).

### REFERENCE EXAMPLE 9

Concentrated hydrochloric acid (128 ml) was added slowly to a suspension of 2-methylsulphenyl-4-nitrotoluene (36.6g) in methanol. Iron powder (36g) was added with stirring whilst maintaining the temperature below 50°C. The mixture was stirred at room temperature for 4 hours. The mixture was poured into water neutralized (by the addition of sodium carbonate), filtered and the residue was extracted with dichloromethane. The aqueous layer was extracted with dichloromethane and the combined organic layers were washed with aqueous sodium chloride solution, dried (anhydrous magnesium sulphate) and filtered. The filtrate was evaporated to dryness and the residue was purified by column chromatography on silica eluted with a mixture of ethyl acetate and n-hexane to give 4-methyl-3-methylsulphenylaniline (12.8g) as an orange solid, NMR (CDCl₃) 2.2(s,3H), 2.35 (s,3H), 3.45 (s,2H), 6.1-6.9 (m,3H).

According to a feature of the present invention, there is provided a method for controlling the growth of weeds (i.e. undesired vegetation) at a locus which comprises applying to the locus a herbicidally effective amount of at least one isoxazole derivative of general formula (I). For this purpose, the isoxazole derivatives are normally used in the form of herbicidal compositions (i.e. in association with compatible diluents or carriers and/or surface active agents suitable for use in herbicidal compositions), for example as hereinafter described.

The compounds of general formula (I) show herbicidal activity against dicotyledonous (i.e. broad-leafed) and monocotyledonous (i.e. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula (I) may be used to control the growth of:
broad-leafed weeds, for example, Abutilon theophrasti, Amaranthus retroflexus Bidens pilosa, Chenopodium album, Galium aparine, Ipomoea spp. e.g. Ipomoea purpurea, Sesbania exaltata, Sinapis arvensis, Solanum nigrum and Xanthium strumarium, and
grass weeds, for example Alopecurus myosuroides, Avena fatua, Digitaria sanguinalis, Echinochloa crus-galli, Sorghum bicolor, Eleusine indica and Setaria spp, e.g. Setaria faberii or Setaria viridis, and
sedges, for example, Cyperus esculentus.

The amounts of compounds of general formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be suff-icient to control the growth of weeds without causing substantial permanent damage to the crop. In general, taking these factors into account, application rates between 0.01kg and 5kg of active material per hectare give good results. However, it is to be understood that higher or lower application rates may be used, depending upon the particular problem of weed control encountered.

The compounds of general formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates between 0.01kg and 4.0kg, and preferably between 0.01kg and 2.0kg, of active material per hectare are particularly suitable.

The compounds of general formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non-directional fashion (e.g. by directional or non-directional spraying) to the weeds or to the soil in which they are expected to appear, before or after planting of the trees or plantations at application rates between 0.25kg and 5.0kg, and preferably between 0.5kg and 4.0kg of active material per hectare.

The compounds of general formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1.0kg and 20.0kg, and preferably between 5.0 and 10.0kg, of active material per hectare are particularly suitable for this purpose.

When used to control the growth of weeds by pre-emergence application, the compounds of general formula (I) may be incorporated into the soil in which the weeds are expected to emerge. It will be appreciated that when the compounds of general formula (I) are used to control the growth of weeds by post-emergence application, i.e. by application to the aerial or exposed portions of emerged weeds, the compounds of general formula (I) will also normally come into contact with the soil and may also then exercise a preemergence control on later-germinating weeds in the soil.

Where especially prolonged weed control is required, the application of the compounds of general formula (I) may be repeated if required.

According to a further feature of the present invention, there are provided compositions suitable for herbicidal use comprising one or more of the isoxazole derivatives of general formula (I), in association with, and preferably homogeneously dispersed in, one or more compatible agriculturally-acceptable diluents or carriers and/or surface active agents [i.e. diluents or carriers and/or surface active agents of the type generally accepted in the art as being suitable for use in herbicidal compositions and which are compatible with compounds of general formula (I)]. The term "homogeneously dispersed" is used to include compositions in which the compounds of general formula (I) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula (I).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl- or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired, herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, talc, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of general formula (I) with solid diluents or by impregnating the solid diluents or carriers with solutions of the compounds of general formula (I) in volatile solvents, evaporating the solvents and, if necessary, grinding the products so as to obtain powders. Granular formulations may be prepared by absorbing the compounds of general formula (I) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of general formula (I) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Unless otherwise specified, the following percentages are by weight. Preferred herbicidal compositions according to the present invention are
aqueous suspension concentrates which comprise from 10 to 70% of one or more compounds of general formula (I), from 2 to 10% of surface-active agent, from 0.1 to 5% of thickener and from 15 to 87.9% of water;
wettable powders which comprise from 10 to 90% of one or more compounds of general formula (I), from 2 to 10% of surface-active agent and from 8 to 88% of solid diluent or carrier;
water soluble or water dispersible powders which comprise from 10 to 90% of one or more compounds of general formula (I), from 2 to 40% of sodium carbonate and from 0 to 88% of solid diluent;
liquid water soluble concentrates which comprise from 5 to 50%, e.g. 10 to 30%, of one or more compounds of general formula (I), from 5 to 25% of surface-active agent and from 25 to 90%, e.g. 45 to 85%, of water miscible solvent, e.g. dimethylformamide, or a mixture of water-miscible solvent and water;
liquid emulsifiable suspension concentrates which comprise from 10 to 70% of one or more compounds of general formula (I), from 5 to 15% of surface-active agent, from 0.1 to 5% of thickener and from 10 to 84.9% of organic solvent;
granules which comprise from 1 to 90%, e.g. 2 to 10% of one or more compounds of general formula (I), from 0.5 to 7%, e.g. 0.5 to 2%, of surface-active agent and from 3 to 98.5%, e.g. 88 to 97.5%, of granular carrier and
emulsifiable concentrates which comprise 0.05 to 90%, and preferably from 1 to 60% of one or more compounds of general formula (I), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula (I) in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled for example alachlor [2-chloro-2,6'-diethyl-N-(methoxy-methyl)-acetanilide], atrazine [2-chloro-4-ethylamino-6-isopropylamino-1,3,5-triazine], bromoxynil [3,5-dibromo-4-hydroxybenzonitrile], chlortoluron [N'-(3-chloro-4-methylphenyl)-N,N-dimethylurea], cyanazine [2-chloro-4-(1-cyano-1-methylethylamino)-6-ethylamino-1,3,5-triazine], 2,4-D [2,4-dichlorophenoxy-acetic acid], dicamba [3,6-dichloro-2-methoxybenzoic acid], difenzoquat [1,2- dimethyl-3,5-diphenylpyrazolium salts], flampropmethyl [methyl N-2-(N- benzoyl-3-chloro-4-fluoroanilino)-propionate], fluometuron [N'-(3-trifluoro-methylphenyl)-N,N-dimethylurea], isoproturon [N'-(4-isopropylphenyl)-N,N-dimethylurea], insecticides, e.g. synthetic pyrethroids, e.g. permethrin and cypermethrin, and fungicides, e.g. carbamates, e.g. methyl N-(1-butyl-carbamoyl-benzimidazol-2-yl)carbamate, and triazoles e.g. 1-(4-chloro-phenoxy)-3,3- dimethyl-1-(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

According to a further feature of the present invention there is provided an article of manufacture comprising at least one of the isoxazole derivatives of general formula (I) or, as is preferred, a herbicidal composition as hereinbefore described, and preferably a herbicidal concentrate which must be diluted before use, comprising at least one of the isoxazole derivatives of general formula (I) within a container for the aforesaid derivative or derivatives of general formula (I), or a said herbicidal composition, and instructions physically associated with the aforesaid container setting out the manner in which the aforesaid derivative or derivatives of general formula (I) or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the isoxazole derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01kg and 20kg of active material per hectare in the manner and for the purposes hereinbefore described.

The following Examples illustrate herbicidal compositions according to the present invention:

### EXAMPLE C1

A soluble concentrate is formed from :

| | |
|---|---|
| Active ingredient (compound A) | 20% w/v |
| Potassium hydroxide solution 33% w/v | 10% v/v |
| Tetrahydrofurfuryl alcohol (THFA) | 10% v/v |
| Water | to 100 volumes. |

by stirring THFA, active ingredient (compound A) and 90% volume of water and slowly adding the potassium hydroxide solution until a steady pH 7-8 is obtained then making up to volume with water.

Similar soluble concentrates may be prepared as described above by replacing the isoxazole (compound A) with other compounds of general formula (I).

### EXAMPLE C2

A wettable powder is formed from :

| | |
|---|---|
| Active ingredient (compound A) | 50% w/w |
| Sodium dodecylbenzene sulphonate | 3% w/w |
| Sodium lignosulphate | 5% w/w |
| Sodium formaldehyde alkylnaphthalene sulphonate | 2% w/w |
| Microfine silicon dioxide | 3% w/w and |
| China clay | 37% w/w |

by blending the above ingredients together and grinding the mixture in an air jet mill.

Similar wettable powders may be prepared as described above by replacing the isoxazole (compound A) with other compounds of general formula (I).

### EXAMPLE C3

A water soluble powder is formed from :

| | |
|---|---|
| Active ingredient (compound A) | 50% w/w |
| Sodium dodecylbenzenesulphonate | 1% w/w |
| Microfine silicon dioxide | 2% w/w |
| Sodium bicarbonate | 47% w/w |

by mixing the above ingredients and grinding the above mixture in a hammer mill.

Similar water soluble powders may be prepared as described above by replacing the isoxazole (compound A) with other compounds of general formula (I).

The compounds of the invention have been used in herbicidal applications according to the following procedures.

### METHOD OF USE OF HERBICIDAL COMPOUNDS:

### a) General

Appropriate quantities of the compounds used to treat the plants were dissolved in acetone to give solutions equivalent to application rates of up to 4000g test compound per hectare (g/ha). These solutions were applied from a standard laboratory herbicide sprayer delivering the equivalent of 290 litres of spray fluid per hectare.

### b) Weed control : Pre-emergence

The seeds were sown in 70 mm square, 75 mm deep plastic pots in non-sterile soil . The quantities of seed per pot were as follows:-

| Weed species | Approx number of seeds/pot |
|---|---|
| 1) Broad-leafed weeds | |
| Abutilon theophrasti | 10 |
| Amaranthus retroflexus | 20 |
| Galium aparine | 10 |
| Ipomoea purpurea | 10 |
| Sinapis arvensis | 15 |
| Xanthium strumarium | 2. |

| 2) Grass weeds | |
|---|---|
| Alopecurus myosuroides | 15 |
| Avena fatua | 10 |
| Echinochloa crus-galli | 15 |
| Setaria viridis | 20. |

| 3) Sedges | |
|---|---|
| Cyperus esculentus | 3. |

| Crop | |
|---|---|
| 1) Broad-leafed | |
| Cotton | 3 |
| Soya | 3. |

| 2) Grass | |
|---|---|
| Maize | 2 |
| Rice | 6 |
| Wheat | 6. |

The compounds of the invention were applied to the soil surface, containing the seeds, as described in (a). A single pot of each crop and each weed was allocated to each treatment, with unsprayed controls and controls sprayed with acetone alone.

After treatment the pots were placed on capillary matting kept in a glass house, and watered overhead . Visual assessment of crop damage was made 20-24 days after spraying. The results were expressed as the percentage reduction in growth or damage to the crop or weeds, in comparison with the plants in the control pots.

### c) Weed control : Post-emergence

The weeds and crops were sown directly into John Innes potting compost in 75 mm deep, 70 mm square pots except for Amaranthus which was pricked out at the seedling stage and transferred to the pots one week before spraying. The plants were then grown in the greenhouse until ready for spraying with the compounds used to treat the plants. The number of plants per pot were as follows :-

### 1) Broad leafed weeds

| Weed species | Number of plants per pot | Growth stage |
|---|---|---|
| Abutilon theophrasti | 3 | 1-2 leaves |
| Amaranthus retroflexus | 4 | 1-2 leaves |
| Galium aparine | 3 | 1^{st} whorl |
| Ipomoea purpurea | 3 | 1-2 leaves |
| Sinapis arvensis | 4 | 2 leaves |
| Xanthium strumarium | 1 | 2-3 leaves. |

### 2) Grass weeds

| Weed species | Number of plants per pot | Growth stage |
|---|---|---|
| Alopecurus myosuroides | 8-12 | 1-2 leaves |
| Avena fatua | 12-18 | 1-2 leaves |
| Echinochloa crus-galli | 4 | 2-3 leaves |
| Setaria viridis | 15-25 | 1-2 leaves |

### 3) Sedges

| Weed species | Number of plants per pot | Growth stage |
|---|---|---|
| Cyperus esculentus | 3 | 3 leaves. |

### 1) Broad leafed

| Crops | Number of plants per pot | Growth stage |
|---|---|---|
| Cotton | 2 | 1 leaf |
| Soya | 2 | 2 leaves. |

### 2) Grass

| Crops | Number of plants per pot | Growth stage |
|---|---|---|
| Maize | 2 | 2-3 leaves |
| Rice | 4 | 2-3 leaves |
| Wheat | 5 | 2-3 leaves. |

The compounds used to treat the plants were applied to the plants as described in (a). A single pot of each crop and weed species was allocated to each treatment, with unsprayed controls and controls sprayed with acetone alone.

After treatment the pots were placed on capillary matting in a glass house, and watered overhead once after 24 hours and then by controlled subirrigation. Visual assessment of crop damage and weed control was made 20-24 days after spraying. The results were expressed as the percentage reduction in growth or damage to the crop or weeds, in comparison with the plants in the control pots.

The compounds of the invention have shown an excellent level of herbicidal activity together with crop tolerance on the weeds used in the foregoing experiments.
When applied pre- or post-emergence at 1000g/ha compounds A, B and C gave at least 90% reduction in growth of one or more of the weed species.

## Claims (Claims for the following Contracting State(s): AT, BE, CH, LI, DE, DK, FR, GB, GR, IT, LU, NL, PT, SE, IE)

1. 4-Benzoyl isoxazole derivatives of general formula (I): 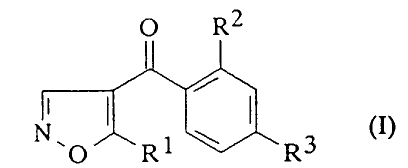 wherein:
R¹ represents cyclopropyl;
R² represents -S(O)ₙR;
R³ represents chlorine, bromine or trifluoromethyl;
R represents methyl; and
n represents 2.

2. A compound according to claim 1 which is 4-(4-chloro-2-methylsulphonylbenzoyl)-5-cyclopropylisoxazole.

3. A compound according to claim 1 which is 5-cyclopropyl-4-(2-methylsulphonyl-4-trifluoromethylbenzoyl)isoxazole.

4. A compound according to claim 1 which is 4-(4-bromo-2-methylsulphonylbenzoyl)-5-cyclopropylisoxazole.

5. A process for the prepararation of a compound according to claim 1 which comprises:
a) the reaction of a compound of general formula (II): 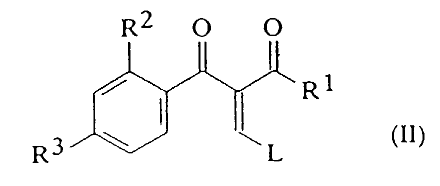 wherein L is a leaving group and R¹, R² and R³ are as defined in claim 1, with a salt of hydroxylamine;
b) the oxidation of compounds of general formula (III): 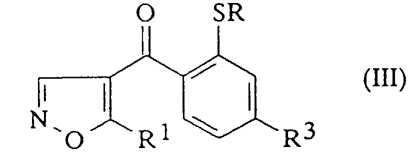 wherein R, R¹ and R³ are as defined in claim 1, to convert the sulphenyl group to a sulphonyl group;
c) the reaction of a compound of general formula (IV): 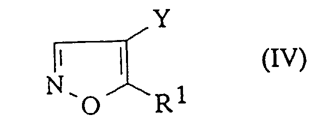 wherein Y represents a carboxy group or a reactive derivative thereof, or a cyano group and R¹ is as defined in claim 1, with an appropriate organometallic reagent.

6. A herbicidal composition which comprises as active ingredient a herbicidally effective amount of an isoxazole derivative of general formula (I) as defined in claim 1 in association with an agriculturally acceptable diluent or carrier and/or surface active agent.

7. A herbicidal composition according to claim 6 which comprises 0.05 to 90% by weight of active ingredient.

8. A herbicidal composition according to claim 6 or 7 which is in liquid form and contains from 0.05 to 25% of surface-active agent.

9. A herbicidal composition according to claim 6, 7 or 8 in the form of an aqueous suspension concentrate, a wettable powder, a water soluble or water dispersible powder, a liquid water soluble concentrate, a liquid emulsifiable suspension concentrate, a granule or an emulsifiable concentrate.

10. A method for controlling the growth of weeds at a locus which comprises applying to the locus a herbicidally effective amount of an isoxazole derivative of general formula (I) as defined in claim 1.

11. A method according to claim 10 in which the locus is an area used, or to be used, for growing of crops and the compound is applied at an application rate from 0.01 kg to 4,0 kg per hectare.

12. A method according to claim 10 in which the locus is an area which is not a crop-growing area and the compound is applied at an application rate from 1,0 kg to 20,0 kg per hectare.

## Claims (Claims for the following Contracting State(s): ES)

1. A process for the preparation of a 4-benzoyl isoxazole derivative of general formula (I): 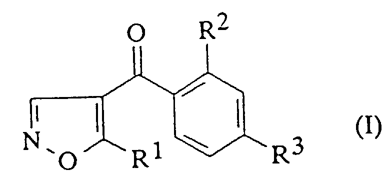 wherein:
R¹ represents cyclopropyl;
R² represents -S(O)ₙR;
R³ represents chlorine, bromine or trifluoromethyl;
R represents methyl; and
n represents 2;
which process comprises:
a) the reaction of a compound of general formula (II): 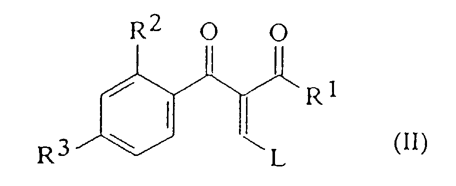 wherein L is a leaving group and R¹, R² and R³ are as hereinbefore defined, with a salt of hydroxylamine;
b) the oxidation of compounds of general formula (III): 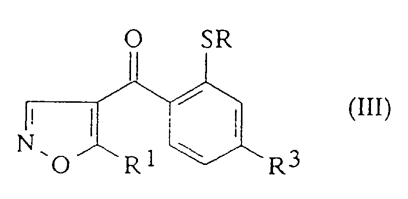 wherein R, R¹ and R³ are hereinbefore defined, to convert the sulphenyl group to a sulphonyl group;
c) the reaction of a compound of general formula (IV): 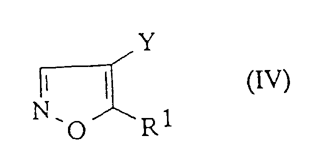 wherein Y represents a carboxy group or a reactive derivative thereof, or a cyano group and R¹ is as hereinbefore defined, with an appropriate organometallic reagent.

2. A process according to claim 1 for the preparation of 4-(4-chloro-2-methylsulphonylbenzoyl)-5-cyclopropylisoxazole.

3. A process according to claim 1 for the preparation of 5-cyclopropyl-4-(2-methylsulphonyl-4-trifluoromethylbenzoyl)isoxazole.

4. A process according to claim 1 for the preparation of 4-(4-bromo-2-methylsulphonylbenzoyl)-5-cyclopropylisoxazole.

5. A process for the preparation of a herbicidal composition which comprises formulating as active ingredient a herbicidally effective amount of an isoxazole derivative of general formula (I) as defined in claim 1 in association with an agriculturally acceptable diluent or carrier and/or surface active agent.

6. A process according to claim 5 for the preparation of a herbicidal composition which comprises 0.05 to 90% by weight of active ingredient.

7. A process according to claim 5 or 6 for the preparation of a herbicidal composition which is in liquid form and contains from 0.05 to 25% of surface-active agent.

8. A process according to claim 5, 6 or 7 for the preparation of a herbicidal composition in the form of an aqueous suspension concentrate, a wettable powder, a water soluble or water dispersible powder, a liquid water soluble concentrate, a liquid emulsifiable suspension concentrate, a granule or an emulsifiable concentrate.

9. A method for controlling the growth of weeds at a locus which comprises applying to the locus a herbicidally effective amount of an isoxazole derivative of general formula (I) as defined in claim 1.

10. A method according to claim 9 in which the locus is an area used, or to be used, for growing of crops and the compound is applied at an application rate from 0.01 kg to 4.0 kg per hectare.

11. A method according to claim 9 in which the locus is an area which is not a crop-growing area and the compound is applied at an application rate from 1.0 kg to 20.0 kg per hectare.

## Patentansprüche (Patentansprüche für folgende(n) Vertragsstaat(en): AT, BE, CH, LI, DE, DK, FR, GB, GR, IT, LU, NL, PT, SE, IE)

1. 4-Benzoyl-isoxazolderivate der allgemeinen Formel (I): 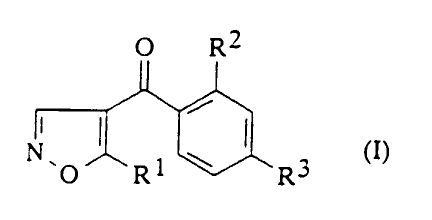 in der
R¹ Cyclopropyl bedeutet,
R² -S(O)ₙR bedeutet,
R³ Chlor, Brom oder Trifluormethyl bedeutet,
R Methyl bedeutet, und
n 2 ist.

2. Verbindung nach Anspruch 1, die 4-(4-Chlor-2-methylsulfonylbenzoyl)-5-cyclopropylisoxazol ist.

3. Verbindung nach Anspruch 1, die 5-Cyclopropyl-4-(2-methylsulfonyl-4-trifluormethylbenzoyl)isoxazol ist.

4. Verbindung nach Anspruch 1, die 4-(4-Brom-2-methylsulfonylbenzoyl)-5-cyclopropylisoxazol ist.

5. Verfahren zur Herstellung einer Verbindung nach Anspruch 1, umfassend:
a) die Umsetzung einer Verbindung der allgemeinen Formel (II): 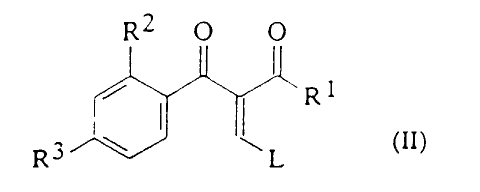 in der L eine austretende Gruppe ist und R¹, R² und R³ wie in Anspruch 1 definiert sind, mit einem Salz von Hydroxylamin;
b) Oxidation von Verbindungen der allgemeinen Formel (III): 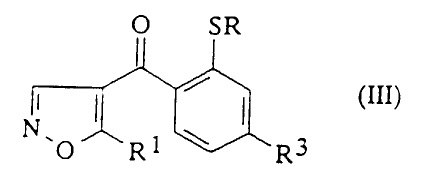 in der R, R¹ und R³ wie in Anspruch 1 definiert sind, zur Umwandlung der Sulfenylgruppe in eine Sulfonylgruppe;
c) Umsetzung einer Verbindung der allgemeinen Formel (IV): 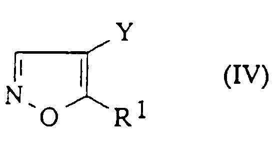 wobei Y eine Carboxygruppe oder ein reaktionsfähiges Derivat davon oder eine Cyanogruppe bedeutet und R¹, wie in Anspruch 1 definiert ist, mit einem entsprechenden metallorganischen Reagenz.

6. Herbizides Mittel, umfassend als Wirkstoff eine herbizid wirksame Menge eines Isoxazolderivates der allgemeinen Formel (I), wie in Anspruch 1 definiert, zusammen mit einem für die Landwirtschaft geeigneten Verdünnungsmittel oder Träger und/oder oberflächenaktivem Mittel.

7. Herbizides Mittel nach Anspruch 6, umfassend 0,05 bis 90 Gew.-% Wirkstoff.

8. Herbizides Mittel nach Anspruch 6 oder 7, das in flüssiger Form vorliegt und 0,05 bis 25 Gew.-% oberflächenaktives Mittel enthält.

9. Herbizides Mittel nach Anspruch 6, 7 oder 8 in Form eines wäßrigen Suspensionskonzentrats, eines netzbaren Pulvers, eines in Wasser löslichen oder in Wasser dispergierbaren Pulvers, eines flüssigen wasserlöslichen Konzentrats, eines flüssigen emulgierbaren Suspensionskonzentrats, eines Granulats oder eines emulgierbaren Konzentrats.

10. Verfahren zur Kontrolle des Wachstums von Unkräutern an einer Stelle, umfassend das Aufbringen einer herbizid wirksamen Menge eines Isoxazolderivats der allgemeinen Formel (I), wie in Anspruch 1 definiert, auf die Stelle.

11. Verfahren nach Anspruch 10, wobei die Stelle eine Fläche ist, die zum Wachstum von Nutzpflanzen verwendet wird oder verwendet werden soll und die Verbindung in einer Aufbringmenge von 0,01 kg bis 4,0 kg/ha aufgebracht wird.

12. Verfahren nach Anspruch 10, wobei die Stelle eine Fläche ist, auf der keine Nutzpflanzen wachsen und die Verbindung in einer Aufbringmenge von 1,0 kg bis 20,0 kg/ha aufgebracht wird.

## Patentansprüche (Patentansprüche für folgende(n) Vertragsstaat(en): ES)

1. Verfahren zur Herstellung eines 4-Benzoylisoxazolderivates der allgemeinen Formel (I): 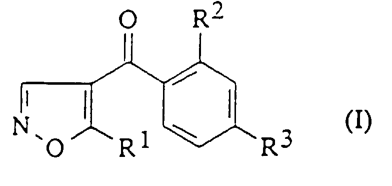 in der
R¹ Cyclopropyl bedeutet,
R² -S(O)ₙR bedeutet,
R³ Chlor, Brom oder Trifluormethyl bedeutet,
R Methyl bedeutet, und
n 2 ist,
wobei das Verfahren umfaßt:
a) die Umsetzung einer Verbindung der allgemeinen Formel (II): 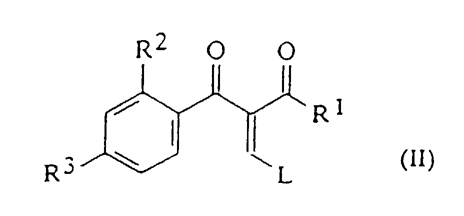 in der L eine austretende Gruppe ist und R¹, R² und R³ wie oben definiert sind, mit einem Salz von Hydroxylamin;
b) Oxidation von Verbindungen der allgemeinen Formel (III): 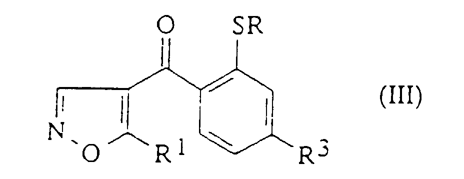 in der R, R¹ und R³ wie oben definiert sind, zur Umwandlung der Sulfenylgruppe in eine Sulfonylgruppe;
c) Umsetzung einer Verbindung der allgemeinen Formel (IV) 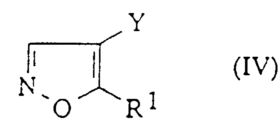 wobei Y eine Carboxygruppe oder ein reaktionsfähiges Derivat davon oder eine Cyanogruppe bedeutet und R¹, wie oben definiert ist, mit einem entsprechenden metallorganischen Reagenz.

2. Verfahren nach Anspruch 1 zur Herstellung von 4-(4-Chlor-2-methylsulfonylbenzoyl)-5-cyclopropylisoxazol.

3. Verfahren nach Anspruch 1 zur Herstellung von 5-Cyclopropyl-4-(2-methylsulfonyl-4-trifluormethyl

4. Verfahren nach Anspruch 1 zur Herstellung von 4-(4-Brom-2-methylsulfonylbenzoyl)-5-cyclopropylisoxazol.

5. Verfahren zur Herstellung eines herbiziden Mittels, umfassend das Zubereiten einer herbizid wirksamen Menge eines Isoxazolderivats der allgemeinen Formel (I), wie in Anspruch 1 definiert, als Wirkstoff zusammen mit einem für die Landwirtschaft verträglichen Verdünnungsmittel oder Träger und/oder einem oberflächenaktiven Mittel.

6. Verfahren nach Anspruch 1 zur Herstellung eines herbiziden Mittels, das 0,05 bis 90 Gew.-% Wirkstoff umfaßt.

7. Verfahren zur Herstellung eines herbiziden Mittels, das in flüssiger Form vorliegt und 0,05 bis 25 % oberflächenaktives Mittel enthält.

8. Verfahren nach Anspruch 5, 6 oder 7 zur Herstellung eines herbiziden Mittels in Form eines wäßrigen Suspensionskonzentrats, eines netzbaren Pulvers, eines in Wasser löslichen oder in Wasser dispergierbaren Pulvers, eines flüssigen wasserlöslichen Konzentrats, eines flüssigen emulgierbaren Suspensionskonzentrats, eines Granulats oder eines emulgierbaren Konzentrats.

9. Verfahren zur Kontrolle des Wachstums von Unkräutern an einer Stelle, umfassend das Aufbringen einer herbizid wirksamen Menge eines Isoxazolderivats der allgemeinen Formel (I), wie in Anspruch 1 definiert, auf die Stelle.

10. Verfahren nach Anspruch 10, wobei die Stelle eine Fläche ist, die zum Wachstum von Nutzpflanzen verwendet wird oder verwendet werden soll und die Verbindung in einer Aufbringmenge von 0,01 kg bis 4,0 kg/ha aufgebracht wird.

11. Verfahren nach Anspruch 10, wobei die Stelle eine Fläche ist, auf der keine Nutzpflanzen wachsen und die Verbindung in einer Aufbringmenge von 1,0 kg bis 20,0 kg/ha aufgebracht wird.

## Revendications (Revendications pour l'(les) Etat(s) contractant(s) suivant(s): AT, BE, CH, LI, DE, DK, FR, GB, GR, IT, LU, NL, PT, SE, IE)

1. Dérivés de 4-benzoylisoxazole, de formule générale (I) : 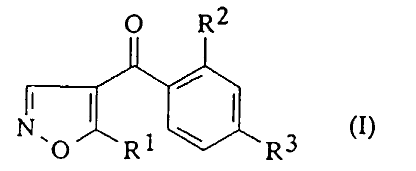 dans laquelle :
R¹ représente un groupe cyclopropyle ;
R² représente un groupe -S(O)ₙR ;
R³ représente le chlore, le brome ou un groupe trifluorométhyle ;
R représente un groupe méthyle ; et
n est égal à 2.

2. Composé suivant la revendication 1, qui consiste en 4-(4-chloro-2-méthylsulfonylbenzoyl)-5-cyclopropylisoxazole.

3. Composé suivant la revendication 1, qui consiste en 5-cyclopropyl-4-(2-méthylsulfonyl-4-trifluorométhylbenzoyl)isoxazole.

4. Composé suivant la revendication 1, qui consiste en 4-(4-bromo-2-méthylsulfonylbenzoyl)-5-cyclopropylisoxazole.

5. Procédé de préparation d'un composé suivant la revendication 1, qui comprend :
a) la réaction d'un composé de formule générale (II) : 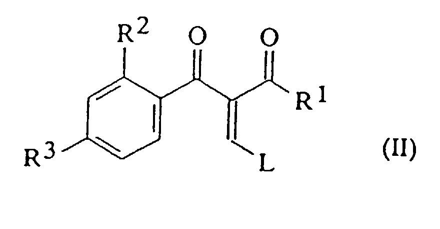 dans laquelle L représente un groupe partant et R¹, R² et R³ répondent aux définitions suivant la revendication 1, avec un sel d'hydroxylamine ;
b) l'oxydation de composés de formule générale (III) : 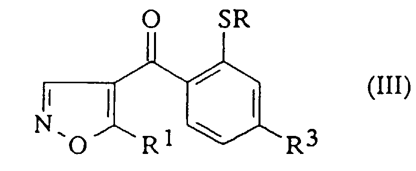 dans laquelle R, R¹ et R³ répondent aux définitions suivant la revendication 1, pour la transformation du groupe sulfényle en un groupe sulfonyle ;
c) la réaction d'un composé de formule générale (IV) : 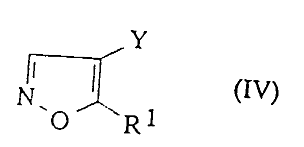 dans laquelle Y représente un groupe carboxy ou un de ses dérivés réactifs, ou un groupe cyano et R¹ répond à la définition suivant la revendication 1, avec un réactif organométallique approprié.

6. Composition herbicide qui comprend comme ingrédient actif une quantité efficace du point de vue herbicide d'un dérivé d'isoxazole de formule générale (I) répondant à la définition suivant la revendication 1 avec un diluant ou support et/ou un agent tensio-actif acceptables en agriculture.

7. Composition herbicide suivant la revendication 6, qui comprend 0,05 à 90 % en poids d'ingrédient actif.

8. Composition herbicide suivant la revendication 6 ou 7, qui est sous forme liquide et qui contient 0,05 à 25 % d'agent tensio-actif.

9. Composition herbicide suivant la revendication 6, 7 ou 8, sous forme d'un concentré de suspension aqueuse, d'une poudre mouillable, d'une poudre hydrosoluble ou dispersable dans l'eau, d'un concentré hydrosoluble liquide, d'un concentré de suspension émulsionnable liquide, de granules ou d'un concentré émulsionnable.

10. Procédé pour lutter contre la croissance de mauvaises herbes dans un milieu, qui comprend l'application à ce milieu d'une quantité efficace du point de vue herbicide d'un dérivé d'isoxazole de formule générale (I) répondant à la définition suivant la revendication 1.

11. Procédé suivant la revendication 10, dans lequel le milieu est un terrain utilisé, ou destiné à être utilisé, pour faire pousser des plantes cultivées et le composé est appliqué à un taux d'application de 0,01 kg à 4,0 kg par hectare.

12. Procédé suivant la revendication 10, dans lequel le milieu est un terrain qui n'est pas destiné à faire pousser des plantes cultivées et le composé est appliqué à un taux d'application de 1,0 kg à 20,0 kg par hectare.

## Revendications (Revendications pour l'(les) Etat(s) contractant(s) suivant(s): ES)

1. Procédé de préparation d'un dérivé de 4-benzoylisoxazole de formule générale (I) : 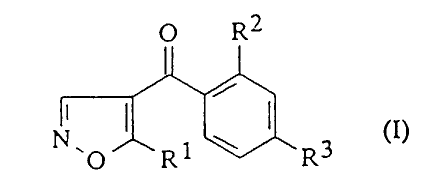 dans laquelle :
R¹ représente un groupe cyclopropyle ;
R² représente un groupe -S(O)ₙR ;
R³ représente le chlore, le brome ou un groupe trifluorométhyle ;
R représente un groupe méthyle ; et
n est égal à 2 ;
procédé qui comprend :
a) la réaction d'un composé de formule générale (II) : 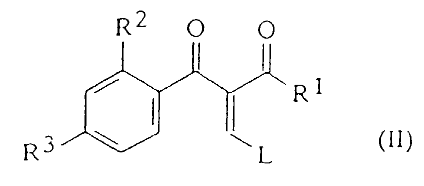 dans laquelle L représente un groupe partant et R¹, R² et R³ répondent aux définitions précitées, avec un sel d'hydroxylamine ;
b) l'oxydation de composés de formule générale (III) : 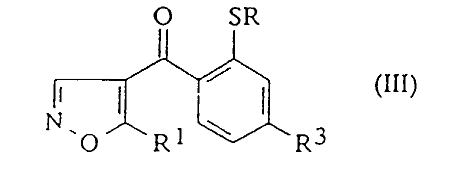 dans laquelle R, R¹ et R³ répondent aux définitions précitées, pour la transformation du groupe sulfényle en un groupe sulfonyle ;
c) la réaction d'un composé de formule générale (IV) : 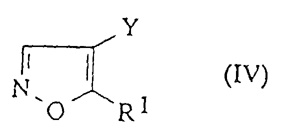 dans laquelle Y représente un groupe carboxy ou un de ses dérivés réactifs, ou un groupe cyano et R¹ répond à la définition précitée, avec un réactif organométallique approprié.

2. Procédé suivant la revendication 1 pour la préparation de 4-(4-chloro-2-méthylsulfonylbenzoyl)-5-cyclopropylisoxazole.

3. Procédé suivant la revendication 1, pour la préparation de 5-cyclopropyl-4-(2-méthylsulfonyl-4-trifluorométhylbenzoyl)isoxazole.

4. Procédé suivant la revendication 1 pour la préparation de 4-(4-bromo-2-méthylsulfonylbenzoyl)-5-cyclopropylisoxazole.

5. Procédé de préparation d'une composition herbicide, qui comprend la formulation, comme ingrédient actif, d'une quantité efficace du point de vue herbicide, d'un dérivé d'isoxazole de formule générale (I) répondant à la définition suivant la revendication 1 en association avec un diluant ou support et/ou un agent tensio-actif acceptables en agriculture.

6. Procédé suivant la revendication 5 pour la préparation d'une composition herbicide qui comprend 0,05 à 90 % en poids d'ingrédient actif.

7. Procédé suivant la revendication 5 ou 6 pour la préparation d'une composition herbicide qui est sous forme liquide et qui contient 0,05 à 25 % d'agent tensio-actif.

8. Procédé suivant la revendication 5, 6 ou 7 pour la préparation d'une composition herbicide sous forme d'un concentré de suspension aqueuse, d'une poudre mouillable, d'une poudre hydrosoluble ou dispersable dans l'eau, d'un concentré hydrosoluble liquide, d'un concentré de suspension émulsionnable liquide, de granules ou d'un concentré émulsionnable.

9. Procédé pour lutter contre la croissance de mauvaises herbes dans un milieu, qui comprend l'application à ce milieu d'une quantité efficace du point de vue herbicide d'un dérivé d'isoxazole de formule générale (I) répondant à la définition suivant la revendication 1.

10. Procédé suivant la revendication 9, dans lequel le milieu est un terrain utilisé, ou destiné à être utilisé, pour faire pousser des plantes cultivées et le composé est appliqué à un taux d'application de 0,01 kg à 4,0 kg par hectare.

11. Procédé suivant la revendication 9, dans lequel le milieu est un terrain qui n'est pas destiné à faire pousser des plantes cultivées et le composé est appliqué à un taux d'application de 1,0 kg à 20,0 kg par hectare.
